# EUROPÄISCHE PATENTANMELDUNG

(11) **EP 0 663 389 A1**
(43) Veröffentlichungstag der Anmeldung: **19.07.1995**
(21) Anmeldenummer: 94120495.0
(22) Anmeldetag: 23.12.1994
(51) Int. Cl.: C07C 213/02, C07C 215/12, C07C 217/28

(54) **Verfahren zur Herstellung von Aminoalkoholen**

(30) Priorität: 12.01.1994 DE 4400591
(71) Anmelder: BASF AKTIENGESELLSCHAFT, D-67056 Ludwigshafen (DE)
(72) Erfinder: Weyer, Hans-Jürgen, Dr., D-67240 Bobenheim-Roxheim (DE); Mercker, Hans Jochen, Dr., D-68239 Mannheim (DE); Becker, Rainer, Dr., D-67098 Bad Dürkheim (DE)

(57) **Zusammenfassung**

Verfahren zur Herstellung von Aminoalkoholen, indem man Hydroxycarbonylverbindungen mit Wasserstoff und Ammoniak oder einem primären oder sekundären Amin bei Temperaturen von 0 bis 300°C und Drücken von 1 bis 400 bar in Gegenwart von Katalysatoren, deren katalytisch aktive Masse aus 50 bis 100 Gew.-% Ruthenium besteht, umsetzt.

## Beschreibung

Die vorliegende Erfindung betrifft ein Verfahren zur Herstellung von Aminoalkoholen aus Hydroxycarbonylverbindungen mit Wasserstoff und Ammoniak oder einem primären oder sekundären Amin in Gegenwart von Katalysatoren, deren katalytisch aktive Masse aus 50 bis 100 Gew-% Ruthenium besteht.

Aus Houben/Weyl, Methoden der organischen Chemie, Bd. XI/1, 4. Aufl., Georg Thieme Verlag, Stuttgart, 1957, Seite 602 bis 648 sind Nickel- oder Kobaltkatalysatoren, vor allem in Form ihrer Raney-Metalle, sowie Platinkatalysatoren zur hydrierenden Aminierung von hydroxy-substituierten Aldehyden bzw. Ketonen bekannt.

US-A-2 016 962 beschreibt die Herstellung von Aminoalkoholen durch Nichtedelmetall-katalysierte, hydrierende Aminierung von Hydroxyaldehyden oder -ketonen. Als Nichtedelmetalle sind Nickel sowie Nickel auf Trägern, jedoch ohne Angaben zu Ausbeuten bekannt.

Raney-Nickel wird beispielsweise für die hydrierende Umsetzung von Glucose mit Methylamin zu N-Methylglucamin (Chem. Abstr., Vol. 82, 140 443q, Ausbeute 85 %), für die analoge Herstellung von N-Ethylglucamin (Chem. Abstr., Vol. 82, 140 441, Ausbeute 85 %) oder die Herstellung von (Aminoalkyl)aminoalkoholen ausgehend von Hydroxycarbonylverbindungen und Diaminoalkanen (DE-A-22 26 869) empfohlen.

Aus der DE-A-28 29 916 ist die hydrierende Aminierung von Dihydroxyaceton bekannt, wobei Raney-Nickel, Pd/C und PtO₂ als Katalysatoren eingesetzt wurden (keine Ausbeuten-Angabe). Weiterhin beschreibt die EP-A-238 961, daß unter den Bedingungen der vorgenannten Anmeldung selbst im Labormaßstab nur Ausbeuten von maximal 60 % erreichbar sind (Seite 1, Zeilen 38 bis 52). Nach der EP-A-238 961 werden bessere Ausbeuten erhalten, wenn man nach einem 2-stufigen Verfahren arbeitet, d.h. erstens Umsetzung der Carbonylverbindung mit dem Amin zum Imin und zweitens katalytische Hydrierung des Imins zum Aminoalkohol. Diese Verfahrensweise ist sehr aufwendig.

Ein zweistufiges Verfahren mit alkoholischen Lösungsmitteln zur Herstellung von N-Methylglucamin ist auch aus der DD-A-23 346 bekannt.

Ebenfalls zweistufige Verfahren werden in WO-A-92/6073, WO-A-92/6984 und WO-A-92/8687 zur Herstellung von Glykaminen beschrieben. Auch hier werden allgemein Nickelkatalysatoren und besonders Nickel-Trägerkatalysatoren eingesetzt. Zur Erreichung eines akzeptablen Schwermetallgehalt im Produkt ist eine komplizierte Vorgehensweise und ein hoher Reaktionsdruck erforderlich. Weiterhin wird das Verfahren dadurch erschwert, daß sich farblose Produkte nur durch eine aufwendige Verfahrensführung erhalten lassen.

Aminoalkohole in guten Ausbeuten und von hoher Reinheit lassen sich nach US-A-2 830 983 durch reduzierende Umsetzung von Hydroxyaldehyden bzw. -ketonen mit Hydrazin erhalten.

Alle diese Vorgehensweisen bringen jedoch erhebliche Nachteile für eine wirtschaftliche Herstellung von Aminoalkoholen mit sich:
Beim Arbeiten in alkoholischer Lösung können nicht die preislich sehr attraktiven wäßrigen Zuckerlösungen eingesetzt werden. Die mit üblichen Katalysatoren erhaltenen Aminoalkohole müssen zur Erzielung hoher Ausbeuten und Produktreinheiten in teuren Verfahren und unter wirtschaftlich nicht optimalen Reaktionsbedingungen synthetisiert werden.

Der vorliegenden Erfindung lag daher die Aufgabe zugrunde, den zuvor genannten Nachteile abzuhelfen.

Demgemäß wurde ein neues und verbessertes Verfahren zur Herstellung von Aminoalkoholen gefunden, welches dadurch gekennzeichnet ist, daß man Hydroxycarbonylverbindungen mit Wasserstoff und Ammoniak oder einem primären oder sekundären Amin bei Temperaturen von 0 bis 300°C und Drücken von 1 bis 400 bar in Gegenwart von Katalysatoren, deren katalytisch aktive Masse aus 50 bis 100 Gew.-% Ruthenium besteht, umsetzt.

Das erfindungsgemäße Verfahren läßt sich wie folgt durchführen:
Die Umsetzung der Hydroxycarbonylverbindungen mit Wasserstoff und Ammoniak oder einem primären oder sekundärem Amin in Gegenwart von Homogen- oder bevorzugt Heterogenkatalysatoren, deren katalytisch aktive Masse aus 50 bis 100 Gew.-%, bevorzugt aus 80 bis 100 Gew.-%, besonders bevorzugt aus 90 bis 100 Gew.-% Ruthenium besteht, kann bei Temperaturen von 0 bis 300°C, bevorzugt 20 bis 200°C, besonders bevorzugt 40 bis 150°C und Drücken von 1 bis 400 bar, bevorzugt 5 bis 300 bar, besonders bevorzugt 10 bis 200 bar in der Gas- oder Flüssigphase diskontinuierlich oder kontinuierlich in Reaktoren wie Rührkesseln oder Rohrreaktoren durchgeführt werden. Wird ein Heterogenkatalysator verwendet, kann dieser als Suspensions- oder Festbettkatalysator eingesetzt werden. Ein Rohrreaktor mit fest angeordnetem Katalysator kann in der Sumpffahrweise oder der Rieselfahrweise betrieben werden.

Alle Einsatzstoffe können in fester, flüssiger oder gasförmiger Form in das erfindungsgemäße Verfahren eingebracht werden.

Das Molverhältnis von Hydroxycarbonylverbindung zum Ammoniak oder primären oder sekundären Amin liegt in der Regel zwischen 1 : 1 und 1 : 50, bevorzugt zwischen 1 : 1,1 und 1 : 10 und besonders bevorzugt zwischen 1 : 1,5 und 1 : 5.

Als Rutheniumkatalysatoren eignen sich beispielsweise Homogen- oder bevorzugt Heterogenkatalysatoren eingesetzt werden. Die Heterogenkatalysatoren können sowohl als Trägerkatalysatoren oder in kompakter Form beispielsweise als RuO₂ z.B. in Form einer Paste eingesetzt werden. Bevorzugt werden Trägerkatalysatoren, wobei die Art des Trägermaterials in der Regel nicht kritisch ist. Es können übliche Trägermaterialien wie Siliciumdioxid, Aluminiumoxide, Zirkondioxid, Titandioxid, Aktivkohle, Silikate oder Zeolithe verwendet werden. Gute Ergebnisse werden mit Aktivkohle, Aluminiumoxid und Zirkondioxid erhalten. Besonders bevorzugt werden Aktivkohle und Aluminiumoxid. Erforderlichenfalls können zur Herstellung der Katalysatoren Bindemittel oder Formhilfsmittel mitverwendet werden. Werden Trägerkatalysatoren eingesetzt, liegt der Rutheniumgehalt üblicherweise zwischen 0,1 und 50 Gew.-%, bevorzugt zwischen 0,2 und 20 Gew.-% und besonders bevorzugt zwischen 0,5 und 10 Gew.-%.

Bezogen auf die Hydroxycarbonylverbindung werden bei der diskontinuierlichen Fahrweise in der Regel 0,0001 bis 20 Gew.-% Ruthenium, bevorzugt 0,01 bis 10 Gew.-% Ruthenium und besonders bevorzugt 0,1 bis 5 Gew.-% Ruthenium eingesetzt.

Die Umsetzung kann in Abwesenheit oder in Gegenwart eines inerten Lösungsmittels durchgeführt werden. Als Lösungsmittel eignen sich beispielsweise Ether, Alkohole oder Wasser. Auch im Überschuß vorhandenes Amin kann als Lösungsmittel dienen. Es können auch Lösungsmittelgemische verwendet werden. Aus Gründen der Wirtschaftlichkeit und Verfügbarkeit sind vor allem bei der hydrierenden Aminierung von Zuckern wasserhaltige Lösungsmittelsysteme bevorzugt. Bei Verwendung von Lösungsmitteln wird die Hydroxycarbonylverbindung in üblichen Konzentrationen, beispielsweise zwischen 1 und 90 Gew.-%, bevorzugt zwischen 10 und 60 Gew.-%, eingesetzt.

Das erfindungsgemäße Verfahren ermöglicht die 1-stufige Synthese von Aminoalkoholen direkt aus Hydroxycarbonylverbindungen und Ammoniak oder primären oder sekundären Aminen in hohen Ausbeuten und Reinheiten. Man kann jedoch auch zuerst nur die Aminierung zu den entsprechenden Schiff'schen Basen bzw. Aminalen und die Hydrierung gegebenenfalls in einem getrennten Reaktor durchführen.

Die Substituenten R¹, R², R³, R⁴, R⁵, R^{1'}, R^{2'}, R^{3'}, R^{4'} und R^{5'} und die Indices n, m, x, y, und z sowie das Zwischenglied X in den Verbindungen I, II und III die folgenden Bedeutungen haben:
R¹, R², R³, R⁴, R⁵, R^{1'}, R^{2'}, R^{3'}, R^{4'}, R^{5'}
- unabhängig voneinander
- Wasserstoff,
- C₁- bis C₂₀-Alkyl,
- C₁- bis C₂₀-Hydroxyalkyl,

R¹ oder R³
- [CH(OH)]ₙ-CH₂-OH

R⁵ 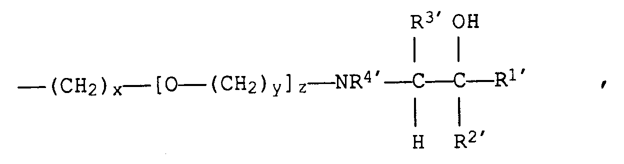 wobei R^{1'}, R^{2'}, R^{3'}, R^{4'}, R^{5'} die unter R¹, R², R³, R⁴, R⁵ genannten Bedeutungen haben,
n, x, y 1 bis 5 und
z eine ganze Zahl von 0 bis 3 wie 0, 1, 2 und 3, bevorzugt 0, 1 und 2, besonders bevorzugt 0 und 1, -C=O und 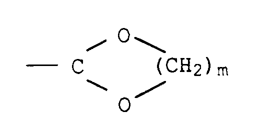
m eine ganze Zahl von 2 bis 6 wie 2, 3, 4, 5 und 6, bevorzugt 2, 3, 4 und 5, besonders bevorzugt 2, 3 und 4.

Als Hydroxycarbonylverbindungen können aliphatische Hydroxyaldehyde und -ketone wie Glycolaldehyd, 1-Hydroxybutanon-2, Saccharide wie Glyceraldehyd, Erythrose, Threose, Ribose Arabinose, Xylose, Lyxose, Allose, Altrose, Glucose, Mannose, Gulose, Idose, Galactose, Talose, Dihydroxyaceton, Erythrulose, Ribulose, Xylulose, Psicose, Fructose, Sorbose, Tagatose, Sucrose, Maltose, Cellobiose, Lactose und Stärke eingesetzt werden. Bevorzugt werden Ribose, Fructose und Glucose, besonders bevorzugt Glucose verwendet. Im besonderen werden Aldosen eingesetzt.

Als Aminkomponente können Ammoniak, primäre Amine wie Methylamin, Ethylamin, t-Butylamin, Dodecylamin, Monoethanolamin, Ethylendiamin, 2,3-Butandiamin, Hexamethylendiamin, 2-(2'-Hydroxyethoxy)ethylamin und sekundäre Amine wie Dimethylamin, Diisopropylamin, Dioctylamin eingesetzt werden. Bevorzugt Ammoniak und primäre Amine, besonders bevorzugt primäre Amine.

Die Aminoalkohole I werden beispielsweise für Kosmetika (JP-A-59/212 419) sowie als Zwischenprodukte bei der Herstellung von nichtionischen Tensiden (EP-A-220 676), Textil-Weichmacher (US-A-3 637 495), Röntgenkontrastmitteln (DD 23 346) und Arzneimittelwirkstoffen (DE-A-28 29 916) verwendet.

### Beispiele

### Verwendete Katalysatoren:

- Katalysator A:: Ru/C (5 Gew.-% Ru), Pulver
- Katalysator B:: RuO₂-Paste
- Katalysator C:: Ru/Al₂O₃ (5 Gew.-% Ru), 1.5 mm-Stränge
- Katalysator D:: Ru/ZrO₂ (5 Gew.-% Ru), 4 mm-Stränge.

Die Katalysatoren B, C und D wurden vor ihrer Verwendung im Wasser-stoffstrom aktiviert.

### Analytik:

Die Proben wurden vor der gaschromatographischen Analyse jeweils durch Umsetzung mit Acetanhydrid/Pyridin acetyliert.

### Beispiel 1

180 g Glucose und 50 g Monomethylamin in 260 ml Wasser wurden zusammen mit 20 g Katalysator A bei 100 bar 4 Stunden bei 60°C hydriert. Der Reaktionsaustrag wurde gaschromatographisch analysiert. Die Ausbeute an N-Methylglucamin betrug 96 %, daneben waren 0,9 % Sorbit entstanden.

### Beispiel 2

In analoger Weise wurden 90 g Glucose und 135 g Dimethylamin in 200 ml Wasser hydriert. Die gaschromatographische Analyse des Reaktionsaustrages ergab eine Ausbeute an N,N-Dimethylglycamin von 95,8 % neben 1,4 % Sorbit und 0,2 % N-Methylglycamin.

### Beispiel 3

90 g Glucose und 112 g Dimethylamin wurden zusammen mit 1 g Katalysator B bei 100 bar 4 Stunden bei 60°C hydriert. Der Reaktionsaustrag wurde gaschromatographisch analysiert. Die Ausbeute an N,N-Dimethylglucamin betrug 95,2 % neben 2,1 % Sorbit.

### Beispiel 4

In einem Rohrreaktor (Länge 200 cm, Durchmesser 16 mm) wurden stündlich 300 g einer 25 gew.-%igen wäßrigen Glucose-Lösung, 100 ml Monomethylamin und 100 l (gemessen unter Normalbedingungen) Wasserstoff an 750 ml Katalysator C bei 200 bar und einer Temperatur in der Reaktormitte von 60°C umgesetzt. Der flüssige Reaktionsaustrag wurde gaschromatographisch untersucht. Die Ausbeute an N-Methylglucamin betrug 95 %, daneben waren 1,3 % Sorbit entstanden.

### Beispiel 5

In einem Rohrreaktor (Länge 200 cm, Durchmesser 16 mm) wurden stündlich 300 g einer 25 gew.-%igen wäßrigen Glucose-Lösung, 300 ml Monomethylamin und 200 l (gemessen unter Normalbedingungen) Wasserstoff an 750 ml Katalysator D bei 200 bar und einer Temperatur in der Reaktormitte von 50°C umgesetzt. Der flüssige Reaktionsaustrag wurde gaschromatographisch untersucht. Die Ausbeute an N-Methylglucamin betrug 96,5 %, daneben waren 0,5 % Sorbit entstanden.

## Patentansprüche

1. Verfahren zur Herstellung von Aminoalkoholen, dadurch gekennzeichnet, daß man Hydroxycarbonylverbindungen mit Wasserstoff und Ammoniak oder einem primären oder sekundären Amin bei Temperaturen von 0 bis 300°C und Drücken von 1 bis 400 bar in Gegenwart von Katalysatoren, deren katalytisch aktive Masse aus 50 bis 100 Gew.-% Ruthenium besteht, umsetzt.

2. Verfahren zur Herstellung von Aminoalkoholen nach Anspruch 1, dadurch gekennzeichnet, daß man als Hydroxycarbonylverbindung Glykolaldehyd, 1-Hydroxybutanon-2, Monosaccharide, Disaccharide, Polysaccharide oder Stärke einsetzt.

3. Verfahren zur Herstellung von Aminoalkoholen der allgemeinen Formel I 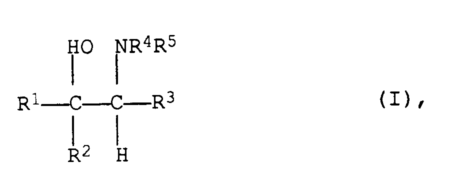 in der
R¹,R²,R³,R⁴,R⁵ Wasserstoff, C₁- bis C₂₀-Alkyl, C₁- bis C₂₀-Hydroxyalkyl,
R¹ oder R³ [CH(OH)]ₙ-CH₂-OH
R⁵ 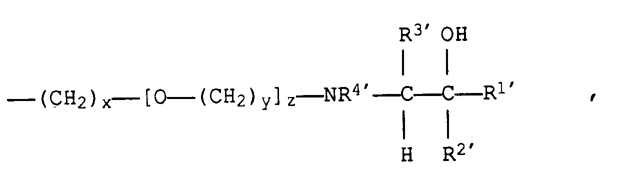 wobei R^{1'}, R^{2'}, R^{3'}, R^{4'}, R^{5'} die unter R¹, R², R³, R⁴, R⁵ genannten Bedeutungen haben,
n, x, y 1 bis 5 und
z 0 bis 3
bedeutet, dadurch gekennzeichnet, daß man Hydroxyverbindungen der allgemeinen Formel II 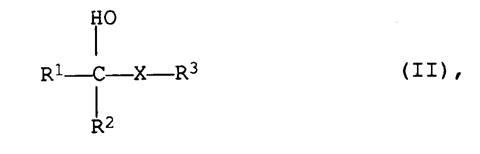 in der R¹, R² und R³ die oben genannten Bedeutungen haben und X für C=O und 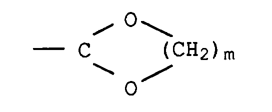 und m für 2 bis 6 steht, mit Wasserstoff und Ammoniak oder einem primären oder sekundären Amin der allgemeinen Formel III 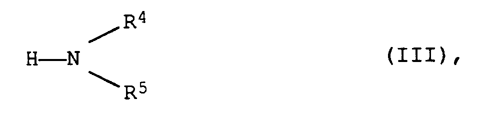 in der R⁴ und R⁵ die oben genannten Bedeutungen haben, bei Temperaturen von 0 bis 300°C und Drücken von 1 bis 400 bar in Gegenwart von Katalysatoren, deren katalytisch aktive Masse aus 50 bis 100 Gew.-% Ruthenium besteht, umsetzt.

4. Verfahren zur Herstellung von Aminoalkoholen nach Anspruch 1, dadurch gekennzeichnet, daß man Katalysatoren einsetzt, deren katalytisch aktive Masse aus 80 bis 100 Gew.-% Ruthenium.

5. Verfahren zur Herstellung von Aminoalkoholen nach Anspruch 1, dadurch gekennzeichnet, daß man Katalysatoren einsetzt, deren katalytisch aktive Masse aus 90 bis 100 Gew.-% Ruthenium besteht.

6. Verfahren zur Herstellung von Aminoalkoholen nach Anspruch 1, dadurch gekennzeichnet, daß man Ruthenium-Trägerkatalysatoren einsetzt.

7. Verfahren zur Herstellung von Aminoalkoholen nach Anspruch 1, dadurch gekennzeichnet, daß man Ruthenium-Trägerkatalysatoren auf Basis Aktivkohle, Aluminiumoxid oder Zirkondioxid einsetzt.

8. Verfahren zur Herstellung von Aminoalkoholen nach Anspruch 1, dadurch gekennzeichnet, daß man Ruthenium-Trägerkatalysatoren mit einem Gehalt von 0,5 bis 10 Gew.-% Ruthenium einsetzt.

9. Verfahren zur Herstellung von Aminoalkoholen nach Anspruch 1, dadurch gekennzeichnet, daß man als Hydroxycarbonylverbindung Glucose einsetzt.

10. Verfahren zur Herstellung von Aminoalkoholen nach Anspruch 1, dadurch gekennzeichnet, daß man Hydroxycarbonylverbindungen kontinuierlich an einem als Festbett angeordneten Ruthenium-Trägerkatalysator mit Wasserstoff und Ammoniak oder einem primären oder sekundären Amin umsetzt.
